# EUROPEAN PATENT SPECIFICATION

(11) **EP 2 547 336 B1**
(45) Date of publication and mention of the grant of the patent: **14.05.2014**
(21) Application number: 11715269.4
(22) Date of filing: 01.03.2011
(51) Int. Cl.: A61K 31/355, A61K 33/06, A61K 33/24, A61K 33/30, A61K 36/47, A61K 36/19, A61K 36/28, A61K 36/87, A61K 36/889, A61K 31/375, A61K 31/4415, A61K 31/455, A61K 31/51, A61K 31/525, A61K 31/714

(54) **A NATURAL SUBSTANCE BASED ANTIOXIDANT AND CHEMOPROTECTIVE AGENT AND ITS METHOD OF PRODUCTION**
AUF EINER NATÜRLICHEN SUBSTANZ BASIERENDES ANTIOXIDATIONS- UND CHEMOPROTEKTIONSWIRKSTOFF UND DESSEN HERSTELLUNGSVERFAHREN
ANTIOXYDANT À BASE DE SUBSTANCE NATURELLE ET AGENT CHIMIOPROTECTEUR, ET PROCÉDÉ DE PRODUCTION ASSOCIÉ

(30) Priority: 18.03.2010 CZ 20100206
(43) Date of publication of application: 23.01.2013
(73) Proprietor: Jahodar, Ludek, 50302 Predmerice nad Labem (CZ); Unibal S.r.o., 37010 Ceske Budejovice (CZ)
(72) Inventor: JAHODAR, Ludek, 50302 Predmerice nad Labem (CZ)
(74) Representative: Novotny, Jaroslav
(86) International application number: PCT/IB2011/050857
(87) International publication number: WO 2011/114249

(56) References cited:
- WO-A1-2006/131932
- US-A1- 2003 104 078
- US-A1- 2008 213 397
- GOPUMADHAVAN S ET AL: "Ameliorative effect of PartySmart in rat model of alcoholic liver disease", INDIAN JOURNAL OF EXPERIMENTAL BIOLOGY, COUNCIL OF SCIENTIFIC & INDUSTRIAL RESEARCH, IN, vol. 46, no. 2, 1 February 2008 (2008-02-01), pages 132-137, XP009148081, ISSN: 0019-5189
- VENKATARANGANNA M V ET AL: "Pharmacodynamics and toxicological profile of PartySmart, a herbal preparation for alcohol hangover in Wistar rats", INDIAN JOURNAL OF MEDICAL RESEARCH, INDIAN COUNCIL OF MEDICAL RESEARCH, INDIA, [Online] vol. 127, no. 5, 1 May 2008 (2008-05-01), pages 460-466, XP009148082, ISSN: 0971-5916 Retrieved from the Internet: URL:www.icmr.nic.in/ijmr/2008/may/0507.pdf > [retrieved on 2011-05-09]

## Description

### Technical Fields

The invention pertains to a natural substance based antioxidant and chemoprotective agent and its method of production whereas the agent contains a dry extract from the fruits of *Vitis vinifera, Emblica officinalis, Phoenix dactylifera*, from the haulms of *Phyllanthus amarus, Andrographis paniculata* and from the entire plant, including the fruit or individual parts of *Cichorium intybus* in the vegetable part while the other, the vitamins-mineral part consists of a mixture of B-group vitamins, vitamin C and E, along with elements Mg, Zn, Mo, Cr, Se.

### Background Arts

A range of herbal agents is used globally containing substances with an antioxidant effect for the purpose of preventing cell damage by oxidative stress. Their effect has a different qualitative and quantitative level. The ingredients used (the form of drug material) and their processing cannot frequently guarantee the bioavailability and homogeneity of the agent. These agents lack fortification of herbal protection of liver tissue in the form of a vitamin-mineral component.

### Disclosure of Invention

According to this invention these disadvantages are removed by the natural substance based antioxidant and chemoprotective agent, the basis of which is that it consists of two components - vegetable and vitamin-mineral. The vegetable component is prepared from dry extracts: from the fruits of *Vitis vinifera*, representing 20 to 60 % of weight of the entire vegetable component, from *Emblica officinalis* 15 to 55 % of weight, from *Phoenix dactylifera* 10 to 55 % of weight, from the haulms of *Phyllanthus amarus* 5 to 20 % of weight, *Andrographis paniculata* from 4 to 20 % of weight of the entire plant or its parts, of *Cichorium intybus* 7 to 20 % of weight. The vitamin-mineral component consists of magnesium 50 to 70 % of weight., the entire vitamin-mineral component, zinc 2.8 to 3.4 % of weight, molybdenum 0.01 to 0.02 % of weight, chromium 0.01 to 0.02 % of weight, selenium 0.01 to 0.02 % of weight, vitamin C 20 to 30 % of weight., vitamin E 3.50 to 4.10 % of weight, pyridoxine 0.40 to 0.50 % of weight, thiamine 0.30 to 0.40 % of weight, riboflavine 0.40 to 0.50 % of weight, nicotinamide 5.0 to 5.50 % of weight, cyanokobalamin 0.0008 to 0.001. % of weight, folic acid 0.06 to 0.07 %. The liquid form of the agent contains 0.015 to 0.04 % of caffeine weight in the final agent.

The method of production of the antioxidant and chemoprotective agent is that each component is homogenised individually and then mixed in proportion: 2 parts of the vegetable component + 1 part of the vitamin-mineral component (converted into active elements), whereas after the final homogenisation, suitable capsules are filled with the solid form of the agent so that the consumer pack does not exceed the daily recommended dosage (DRD) or is adjusted in a different suitable solid form while maintaining the same conditions for DRD, whereas the liquid form arises after the solid form has been dissolved in the required amount of water creating a solution of 0.15 to 0.3%, with appropriate contents of 0.015 to 0.04 % of the caffeine weight in the final agent.

Its application as an antioxidant and chemoprotective mixture is connected with the creation of reactive (toxic) form of oxygen (ROS) and free radicals (FR) whose role in the pathogenesis of tissue changes is proved. As an example, we can mention the effect of alcohol on hepatocyte microsomes where the hydroxyethyl free radical has been proved. This, along with ROS being endogenously produced by endoplasmic reticulum, is increased by the cytochrome P450 isoenzym catalysing the transformation of ethanol. Isoenzym can trigger oxidative damage in chronic alcohol intoxications. As far as acute intoxication is concerned, it is likely that the increased amount of acetaldehyde in hepatocyte cytosol is oxidized by alternative ways under catalysis by xanthinoxidase and aldehydoxidase while producing superoxide radical at the same time. The burden of ethanol on the liver is accompanied partly by an increased occurrence of ROS, free radicals and by a decrease of antioxidant protection. Thus, ethanol intoxication is able to induce oxidating stress by a change in the proportion between the prooxidation and antioxidation reactions.

### Made for Carring out the Invention

The natural-based antioxidant and chemoprotective agent consists of a vegetable component which is prepared from dry extracts: from the fruits of *Vitis vinifera* representing 30 % of weight of the entire vegetable component, from *Emblica officinalis* 20 % of weight, from *Phoenix dactylifera* 18 % of weight, from the haulms of *Phyllanthus amarus* 20 % of weight, *Andrographis paniculata* from 5 % of weight of the entire plant or its parts, from *Cichorium intybus* 7 % of weight and supplemented by a vitamin-mineral component consisting of magnesium 58.20 % of weight, zinc 3.40 % of weight, molybdenum 0.017 % of weight, chromium 0.014 % of weight, selenium 0.018 % of weight, vitamin C 27.37 % of weight, vitamin E 4.10 % of weight, pyridoxine 0.48 % of weight, thiamine 0.38 % of weight, riboflavine 0.48 % of weight, nicotinamide 5.47 % of weight, cyanokobalamin 0.001 % of weight and folic acid 0.07 % of weight.

The natural substance based anti-oxidative and chemoprotective agent is produced in such a way that each component is homogenised individually and then mixed in proportion to the vegetable component - 2 parts + the vitamin-mineral component - 1 part (the mineral component weight depends on the compounds used, being recalculated into effective elements). After the final homogenisation, suitable capsules are filled with the solid form of the agent so that the consumer pack does not exceed the daily recommended dosage (DRD) or is adjusted in a different suitable solid form while maintaining the same condition. The liquid form arises after the solid form has been dissolved in the required amount of water so that there is a solution of 0.15 to 0.3%. The liquid form is enriched by 0.025 % of the caffeine weight in the final agent.

In order to prepare 1000 daily recommended doses of the final solid agent according to the invention using the permitted forms of vitamins and mineral substances, 600 g of the vegetable component is mixed following procedure A and 1 134 g of vitamin-mineral component following procedure B. It is additionally homogenised and divided into 1 000 recommended doses. The recommended daily dose in the mentioned example of 1734 mg is made up, for instance, in 2 to 4 capsules, 2 to 3 tablets, in split pill etc. depending on the selected form of dosage.

### Procedure A

The following is mixed and homogenised:
180 g of a dry aqueous extract from the fruits (3:1) of *Vitis vinifera*
120 g of a dry aqueous -ethanol extract from the fruits (4:1) of *Emblica officinalis*
108 g of a dry aqueous extract from the fruits (2:1) of *Phoenix dactylifera*
120 g of a dry aqueous -ethanol extract from the haulm( (10:1.) *Phyllanthus amarus*
30 g of a dry aqueous extract from the haulm (10:1) *Andrographis paniculata*
42 g of a dry aqueous extract from the root (10:1) of *Cichorium intybus (600 mg of the vegetable part in one RDP)*

### Procedure B

The following is mixed and homogenised:
1000 g of anhydrous magnesium acetate (corresponding to 170 mg of Mg)
   21 g of zinc chloride (corresponding to 10 mg of Zn)
   0. 107 g of anhydrous sodium molybdate (corresponding to 50 µg of Mo)
   0.280 g of chromium sulphate octadecahydrate (corresponding to 40 µg Cr)
   0.183 g of sodium selentite pentahydrate (corresponding to 55 µg of Se)
80 g of vitamin C
12 g of vitamin E
1. 1 g of vitamin B 1
1. 4 g of vitamin B2
16 g of nicotinamide (B3)
1. 4 g of vitamin B6 (pyridoxine)
0. 200 g of folic acid
0. 0025 g of vitamin B 12
*(292.25 mg of efficient components of the vitamin-mineral component in one RDP)*

### Description of the individual components and their functions

The proposed composition of the agent is suitable for the prescribed application because of the characteristics mentioned below of the content substances of individual extracts of the vegetable component and of vitamins and mineral substances forming the other component.

The dry extract, originally aqueous, most frequently 3:1, from the fruits of the grapevine (*Vitis vinifera*) contains biologically active polyphenolic compounds of the type of flavonoids, antokyans, cynnamic acid derivatives and others with distinctive anti-ROS (effect against reactive/toxic forms of oxygen.) This fact has been proved by many pharmacological studies. Adverse effects and interactions are hypothetical only.

The dry extract, originally aqueous -ethanol, most frequently 4:1, from the fruits of Embelic Myrobalans (*Emblica officinalis*), contains citric acid, ascorbic acid, Gallic acid and its derivatives, further catechins, flavonoids and other substances with significant anti-ROS activity. Specialized literature also describes the hypocholesterolemic and hypolipidemic effects. The fruits are used as fruit, being a part of the traditional medicine in the countries of origin. Adverse effects and interactions are not described.

The dry extract, originally aqueous, from the fruits of the date palm (*Phoenix dactylifera*) most frequently 2:1 is prepared from common fruit (dates). It is an entirely safe raw ingredient in portions not exceeding the equivalent of fruit consumption. The fruits contain sugars, vitamin B1 and pro-vitamin A.

The dry extract, originally aqueous-ethanol, most frequently 10:1, from the haulm of *Phyllanthus amarus*, contains amariin, a hydrolysable tanning substance, quercetine type flavonoids and lignans. Traditional medicine uses the drug for preventive protection of liver and kidney tissue. These effects have been confirmed also by trustworthy current pharmacological studies; in addition a positive effect on the eradication of hepatitis B virus is confirmed. The drug and the extract made from this could, in high doses, influence the glycemia in DMT1 up to a hyperglycemia state. The drug is completely safe administered in the DRD (daily recommended dosage) of the agent.

The dry extract, originally ethanol, most frequently 10:1, from the haulm of King of Bitters (*Andrographis paniculata*), contains biologically active andrographolide diterpenic lactones, labdenic diterpenic lactones, prenylated sesquiterpen lactones etc. The chemoprotective effect on liver tissue is experimentally confirmed. There is a more significant effect than when using silymarine. The other proved effects are: immunostimulating, analgetic, antipyretic, anti-aggregation, anti-HIV. The adverse effects at high doses are described as being gastrointestinal distress, anorexia and emesis. Interaction with drugs theoretical only: increase of the antiaggregation effect in anticoagulations and interference with imunosupressives. The concentration of substances being demonstrated by adverse effects is manifoldly lower than as stated in the experiments.

The dry extract, originally aqueous, most frequently 10:1, from different parts of chicory (*Cichorium intybus*), usually contains polysaccharide inulin, sesquiterpen lactones, caffeic acid derivatives, coumarins and flavonoids, chemoprotective flavonolignans and others. Interaction and adverse effects are not described.

Mineral substances - biogenous elements represented in the agent are cofactors of enzymes; they are a part of enzymatic active centres playing a significant role in a biotransformation process of xenobiotics (detoxication and elimination reactions).

Magnesium (Mg) will be represented in the agent in the form stated in the valid Ministry of National Health decree, in the amount not exceeding the DRD stated in the decree. Magnesium is an essential element of tissues and body fluids, it is an important enzyme activator in all reactions in which trinucleotides participate, most frequently ATP. It partakes in the biosynthesis of DNA and RNA.

Zinc (Zn) will be represented in the agent in the form stated in the valid Ministry of National Health decree, in the amount not exceeding the DRD stated in the decree. Zinc is an essential trace element, being a part of carbonate dehydratase and carboxypeptidases. There is insulin zinc suspension in the islets of Langerhans. It partakes in reactions catalyzed by glumate dehydrogenase, alcohol dehydrogenase and kidney phophatase.

Chromium (Cr) will be represented in the agent in the form stated in the valid Ministry of National Health decree, in the amount not exceeding the DRD stated in the decree. Chromium is an essential trace element, partaking in the transformation of saccharides and lipids, potentiating the insulin effect.

Molybdenum (Mo) is an essential trace element, it will be represented in the agent in the form stated in the valid Ministry of National Health decree, in the amount not exceeding the DRD stated in the decree. It is a part of xantin-oxidase and other flavoproteins.

Selenium (Se) is a trace element, it will be represented in the agent in the form stated in the valid Ministry of National Health decree, in the amount not exceeding the DRD stated in the decree. Selenium is a part of selenium-proteins with enzymatic functions (glutathionperoxidase), it provides protection against reactive forms of oxygen.

### Vitamins

Most of the vitamins used (especially from the B group) constitute precursors of coenzymes, others are the direct reactants of biochemical transformations.

Vitamin C (ascorbic acid) will be represented in the agent in the form stated in the valid Ministry of National Health decree, in the amount not exceeding the DRD stated in the decree. The substance is a significant biological antioxidant, making the immune system and other body systems more effective.

Vitamin E (tocopherol) will be represented in the agent in the form stated in the valid Ministry of National Health decree, in the amount not exceeding the DRD stated in the decree. The vitamin is a membrane antioxidant, having a significant FRS activity (free radicals scavenger) and thereby also a chemoprotective effect.

Vitamin B 1 (thiamine) will be represented in the agent in the form stated in the valid Ministry of National Health decree, in the amount not exceeding the DRD stated in the decree. The substance is significant for the activity of nervous tissue and sugar metabolism; it is a coenzyme of enzymes, especially of those participating in oxidative decarboxylation of keto acids.

Vitamin B2 (riboflavine) will be represented in the agent in the form stated in the valid Ministry of National Health decree, in the amount not exceeding the DRD stated in the decree. A vitamin which plays a significant role in cell breathing is the coenzyme FAD.

Vitamin B3 (niacin, nicotinamide) will be represented in the agent in the form stated in the valid Ministry of National Health decree, in the amount not exceeding the DRD stated in the decree. The coenzyme of a range of enzymes (NAD) participating in a lot of metabolic processes, especially oxidoreduction processes.

Vitamin B6 (pyridoxine), a range of enzymes. Coenzym of transaminases and decarboxylases.

Vitamin B12 (cyanokobalamin) will be represented in the agent in the form stated in the valid Ministry of National Health decree, in the amount not exceeding the DRD stated in the decree. A significant growth factor which significantly influences the renewal of cells in all organs, especially then in nervous tissue and during hematopoiesis.

Folic acid, will be represented in the agent in the form stated in the valid Ministry of National Health decree, in the amount not exceeding the DRD stated in the decree. It is a precursor of the coenzyme of the enzymes partaking in the transformations of homocysteine, serine, histidine, purine synthesis thymidylate and others.

## Claims

1. A natural substance based antioxidant and chemoprotective agent, **characterised by** the fact, that it comprises two parts of the vegetable component, which is prepared from dry extracts from the fruits of *Vitis vinifera*, representing 20 to 60 of weight % of this component, by a dry extract from the fruits of *Emblica officinalis*, representing 15 to 55 % of weight, by a dry extract from the fruits of *Phoenix dactylifera* representing 10 to 55 % of weight, by a dry extract from the entire plant or individual parts *Cichorium intybus* representing 7 to 20 % of weight by a dry extract from the haulm of *Andrographis paniculata* representing 4 to 20 % of weight and a dry extract from the haulm of *Phyllanthus amarus* representing 5 to 20 % of weight and one part of the vitamin-mineral component, converted into efficient elements consisting of magnesium 50 to 70% of weight, zinc 2.8 to 3.4 % of weight, molybdenum 0.01 to 0.02 % of weight, chromium 0.01 to 0.02 % of weight, selenium 0.01 to 0.02 % of weight, vitamin C 20 to 30 % of weight, vitamin E 3.50 to 4.10 % of weight, thiamine 0.30 to 0.40% of weight, riboflavine 0.40 to 0.50 % of weight, pyridoxine 0.40 to 0.50 % of weight, nicotinamide 5 to 5.50 % of weight and folic acid 0.06 to 0.07 % of weight, cyanokobalamin 0.0008 to 0.001 % of weight.

2. The natural substances based antioxidant and chemoprotective agent according to Claim 1, **characterised by** the fact, that a liquid form of the agent contains 0.015 to 0.04 % of the caffeine weight in the final agent.

3. The method of the production of the agent according to Claim 1 or 2 **characterised by** the fact, that each component is homogenised individually and then mixed in proportion to the vegetable component 2 parts + 1 part of the vitamin-mineral component (recalculated into active elements) whereas after the final homogenisation, suitable capsules are filled with the solid form of the agent so that the consumer pack does not exceed the daily recommended doses (DRD) or is adjusted in a different suitable solid form while maintaining the same conditions for DRD optionally wherein the liquid form arises after the solid form has been dissolved in the required amount of water so that a solution of 0.15 to 0.3% of the weight arises with an appropriate content of 0.015 to 0.04 % of the caffeine weight in the final agent.

## Patentansprüche

1. Natürliches Mittel auf der Basis der antioxidativen und chemoprotektiven Stoffe, **dadurch gekennzeichnet, dass** es aus zwei Komponenten besteht, indem der vegetabile (Kräuter-)Komplex aus dem getrockneten Extrakt der Früchte *Vitis vinifera,* das 20 bis 60 Gewichtsprozent (Massenanteil) dieser Komponente darstellt, aus dem getrockneten Extrakt der Früchte *Emblica officinalis,* das 15 bis 55 Gewichtsprozent darstellt, aus dem getrockneten Extrakt der Früchte *Phoenix dactylifera,* das 10 bis 55 Gewichtsprozent darstellt, aus dem getrockneten Extrakt der ganzen Pflanze oder deren Teile *Cichorium intybus,* das 7 bis 20 Gewichtsprozent darstellt, aus dem getrockneten Extrakt des Blattwerks *Andrographis* paniculata, das 4 bis 20 Gewichtsprozent darstellt, und aus dem getrockneten Extrakt des Krautwerks *Phyllanthus amarus*, das 5 bis 20 Gewichtsprozent dieser Komponente darstellt, zubereitet wird, wobei die zweite Komponente der Vitamin-Mineralkomplex mit den wirksamen Elementen: Magnesium, mit 50 bis 70 Gewichtsprozent, Zink mit 2.8 bis 3.4 Gewichtsprozent, Molybdän mit 0.01 bis 0.02 Gewichtsprozent, Chrom mit 0.01 bis 0.02 Gewichtsprozent, Selen mit 0.01 bis 0.02 Gewichtsprozent, das Vitamin C mit 20 bis 30 Gewichtsprozent, Vitamin E mit 3.50 bis 4.10 Gewichtsprozent, Thiamin mit 0.30 bis 0.40 Gewichtsprozent, Riboflavin mit 0.40 bis 0.50 Gewichtsprozent, Pyridoxine mit 0.40 bis 0.50 Gewichtsprozent, Nicotinamid mit 5 bis 5.50 Gewichtsprozent, Folsäure mit 0.06 bis 0.07 Gewichtsprozent, Zyanokobalamin (Vit. B12) mit 0.0008 bis 0.001 Gewichtsprozent, ist

2. Die Naturstoffe auf der Basis der antioxidativen und chemoprotektiven Stoffe gemäß Anspruch 1, **dadurch gekennzeichnet, dass** die flüssige Komponente des Mittels 0.015 bis 0.04 Gewichtsprozent Koffein in der Finalzubereitung enthält.

3. Das Herstellungsverfahren des Präparates gemäß Anspruch 1 und 2., **dadurch gekennzeichnet, dass** jede Komponente selbständig homogenisiert wird, wobei die einzelnen Komponenten anschließend im Verhältnis 2 Teile der vegetabilen (Kräuter-)Komponente + 1 Teil der Vitamin-Mineralkomponente gemischt (umgerechnet auf aktive Elemente) werden, wobei die Kapseln nach der Finalhomogenisierung mit der festen Form der Zubereitung so aufgefüllt werden, dass die Verbraucherpackung die empfohlenen Tagesdosen (RDA) nicht überschreitet, oder die Zubereitung in eine andere feste Form bei Aufrechterhaltung derselben Bedingungen für RDA gebracht wird, indem ggf. die flüssige Komponente zugegeben wird, nachdem die feste Form der Zubereitung in der erforderlichen Wassermenge so aufgelöst wird, dass die Lösung, die 0.15 bis 0.3 Gewichtsprozent darstellt, den entsprechenden Koffeingehalt von 0.015 bis 0.04 Gewichtsprozent im Finalprodukt ergibt.

## Revendications

1. Agent naturel basé sur des substances antioxydantes et chimioprotectives constitué de deux composantes - composante végétale (plantes officinales) préparée à partir d'un extrait séché des fruits de *Vitis vinifera,* représentant 20 à 60 %m de cette composante, d'un extrait séché des fruits de *Emblica officinalis,* représentant 15 à 55 %m, d'un extrait séché des fruits de *Phoenix dactylifera*, représentant 10 à 55 %m, d'un extrait séché de la plante entière ou des parties de *Cichorium intybus,* représentant 7 à 20 %m, d'un extrait séché de la fane de *Andrographis* paniculata, représentant 4 à 20 %m et d'un extrait séché de la fane de *Phyllanthus amarus*, représentant 5 à 20 %m ce cette composante, la deuxième composante étant formée d'un complexe de vitamines et de substances minérales avec des éléments actifs, dont le magnésium, représentant 50 à 70 %m, le zinc avec 2,8 à 3,4 %m, le molybdène avec 0,01 à 0,02 %m, le chrome avec 0,01 à 0,02 %m, le sélénium avec 0,01 à 0,02 %m, la vitamine C avec 20 à 30 %h, la vitamine E avec 3,50 à 4,10 %m, la thiamine avec 0,30 à 0,40 %m, la riboflavine avec 0,40 à 0,50 %m, la pyridoxine avec 0,40 à 0,50 %m, la nicotinamide avec 5 à 5,50 %m, l'acide folique avec 0,06 à 0,07 %m, la cyanocobalamine (vitamine B12) avec 0,0008 à 0,001 %m.

2. Substances naturelles avec des effets antioxydants et chimio-protectrices conformément selon les revendications 1 **caractérisées par le fait que** la composante liquide contient 0,015 à 0,04 %m de caféine dans la préparation.

3. La méthode de fabrication de la préparation selon les revendications 1 et 2 se **caractérise par le** f a i t que chaque composante est homogénéisée indépendamment et après les deux composantes sont mélangées dans une proportion de 2 parts de composante végétale pour 1 part du complexe de vitamines et de substances minérales (recalculé par rapport aux éléments actifs), alors qu'après l'homogénéisation finale, les capsules sont remplies de forme solide de la préparation de manière à ne pas dépasser les apports nutritionnels conseillés (ANC) dans l'emballage-consommateur, ou d'une autre forme solide adéquate conservant les mêmes conditions pour l'ANC, le cas échéant, on ajoute la composante liquide après la dissolution de la forme solide de la préparation dans une quantité d'eau requise de manière à ce que la solution représentant 0,15 à 0,3 %m représente une quantité de 0,015 à 0,04 % de caféine dans le produit final.
